# EUROPEAN PATENT APPLICATION

(11) **EP 1 591 160 A2**
(43) Date of publication of application: **02.11.2005**
(21) Application number: 05251250.6
(22) Date of filing: 02.03.2005
(51) Int. Cl.: B01J 19/00, C12Q 1/68, C07H 21/00, C07K 1/04, C07B 61/00

(54) **Releasable polymer arrays**

(30) Priority: 02.03.2004 US 791005
(71) Applicant: Affymetrix, Inc., Santa Clara, CA 95051 (US)
(72) Inventor: Cuppoletti, Andrea, Livermore, CA 94550 (US); McGall, Glenn H., Palo Alto, CA 94303 (US)
(74) Representative: Bizley, Richard Edward

(57) **Abstract**

Methods are provided for fabricating an array of polymers wherein the polymers may be released from the surface of the array by activation of a cleavable moiety. Also provided are arrays of polymers wherein the polymers can be released from the surface of the array by activation of a releasable group. Arrays of nucleic acids wherein a nucleic acid probe may be released from the array by activation of a releasable groups and methods for fabrication of such arrays are also disclosed.

## Description

### PRIORITY CLAIM

This application claims priority to U.S. Application Serial No. 10/791,005, filed March 2, 2004, which is a continuation in part of U.S. Application Serial No. 10/738,381, filed December 16, 2003 which claims priority to U.S. Provisional Application Serial No. 60/434,144 filed on December 17, 2002, which is incorporated herein referenced in its entirety.

### RELATED APPLICATIONS

This application is related to U.S. Application Serial No. 10/272,155 filed on October 14, 2002, which is incorporated herein referenced in its entirety.

### FIELD OF THE INVENTION

The present invention relates generally to the field of polymer arrays. More specifically, the present invention relates to the release of arrays of polymers bound to a solid substrate through a linker, wherein the linker comprises a cleavable moiety which is activatable or cleavable under a distinct set of conditions, e.g., particular wavelengths of electromagnetic radiation, chemical conditions (acidic, basic, etc.), certain electric current or field conditions.

### BACKGROUND OF THE INVENTION

U.S. Pat. No. 5,424,186 to Fodor, et al., describes a technique for, among other things, forming and using high density arrays of probes comprising molecules such as oligonucleotide, RNA, peptides, polysaccharides, and other materials. Arrays of oligonucleotides or peptides, for example, are formed on the surface by sequentially removing a photo-removable group from a surface, coupling a monomer to the exposed region of the surface, and repeating the process. Nucleic acid probe arrays synthesized in this manner, such as Affymetrix GeneChip® probe arrays from Affymetrix, Inc. of Santa Clara, Calif. have been used to generate unprecedented amounts of information about biological systems. Analysis of these data may lead to the development of new drugs and new diagnostic tools.

A typical step in the process of synthesizing these probe arrays is to design a mask that will define the locations on a substrate that are exposed to light. Some systems and methods useful in the design and/or use of such masks are described in the following U.S. Pat. Nos. 5,571,639 to Hubbell, et al.; 5,593,839 to Hubbell, et al.; 5,856,101 to Hubbell, et al.; 6,153,743 to Hubbell, et al.; and 6,188,783 to Balaban, et al., each of which is hereby incorporated herein by reference for all purposes.

The present invention relates to release of polymers, including nucleic acid probes, from an array.

### SUMMARY OF THE INVENTION

Methods are provided for releasing polymers from an array of polymers. One disclosed method has the steps of providing a solid substrate; attaching a plurality of linkers to the substrate, each said linker having a cleavable moiety, wherein the cleavable moiety is activatable at a distinct set of conditions and wherein activation of said cleavable moiety disrupts the linker to allow release of the polymer, to provide a plurality of attached linkers; attaching a first monomer to at least one of said plurality of linkers to provide an attached first monomer; attaching a second monomer to a least one of said attached first monomers or said attached plurality of polymers to provide an attached second monomer; attaching a third monomers to a least one of said attached first monomer, second monomers or plurality of linkers to provide an attached third monomer; repeating said step of attaching a monomer until the desired array of polymers is complete and subjecting the array to the distinct set of conditions to provide release of polymers from said array.

In another disclosed method the steps are: attaching a plurality of linkers to a solid substrate having a surface, the linker comprising a cleavable moiety, wherein the cleavable moiety is activatable at a distinct set of conditions and wherein activation of the cleavable moiety disrupts the linker to allow release of the polymer from the array, to provide a plurality of attached linkers and wherein each linker has two terminal ends, the first end of which is attached to the substrate and the second end of which is away from the substrate and comprises a reactive group covered by a photoprotective removable group having a first activation energy wavelength; selectively exposing said photoprotective group on the attached linkers to light to selectively remove said photoprotective group and expose said reactive group in predefined regions to provide a deprotected array; exposing under reactive conditions the deprotected reactive groups to a first monomer and attaching the first monomer to an exposed reactive group, wherein the first monomer comprises a reactive group protected by a photoprotective removable group having the first activation energy wavelength; selectively exposing said photoprotective removable groups on said attached linkers or on said first attached monomer to light to selectively remove said photoprotective groups and expose said reactive groups in predefined regions; exposing under reactive conditions the deprotected reactive groups to a second monomer and attaching second monomers to exposed protective groups, wherein the second monomer comprises a reactive group protected by a photoprotective removable group having the first activation energy wavelength; selectively exposing said photoprotective group on said attached linkers or said first monomers to light to selectively remove the photoprotective groups and expose the reactive groups in predefined regions; exposing the deprotected groups to a second monomer and attaching second monomers to exposed reactive groups, wherein the second monomer comprises a reactive group protected by a photoprotective removable group having the first activation energy wavelength; repeating the steps of deprotecting exposed reactive groups and of attaching further monomers until the desired array of polymers is complete and subjecting the array to the distinct set of conditions to release the array of polymers.

Arrays of releasable polymers are provided, the array comprising a solid substrate having a linker comprising a releasable group which is cleavable under a distinct set of conditions and attached to said linker a polymer, wherein the polymer can be released by exposure of the array to the distinct set of conditions.

The present invention also discloses polymer arrays having a releasable nucleic acid probe, the polymer array comprising a solid substrate having attached thereto a polymer, at least one of the polymers comprising a cleavable moiety which is labile under a distinct set of conditions wherein the cleavable moiety allows release of the polymer upon activation.

Also provided are methods for fabricating a polymer array having releasable polymers, the method having the steps of: providing a substrate; attaching a linker to the substrate, the linker comprising a cleavable moiety which is labile under a distinct set of conditions; reversibly modifying the cleavable moiety with a protecting group to provide a reversibly modified releasable group wherein the modified releasable group is not labile under the distinct set of conditions; attaching a first monomer to the linker; attaching a second monomer to the linker or to the first monomer; repeating the step of attaching a further monomer until the desired polymer array is provided; and demodifying the reversibly modified releasable group.

### DETAILED DESCRIPTION OF THE INVENTION

### DEFINITIONS

The following definitions are set forth to illustrate and define the meaning and scope of the various terms used to describe the invention herein.

The terms "solid substrate" and "solid support" are used interchangeably herein and refer to the bulk, underlying, and core material which can contain additional layers of material. The solid support is a material having a rigid or semi-rigid surface. Such materials preferably take the form of plates or slides, small beads, pellets, disks or other convenient forms, although other forms can also be used. In some embodiments, at least one surface of the substrate is substantially flat. In other embodiments, a roughly spherical shape is preferred. The solid support can be biological, nonbiological, organic, inorganic, or a combination of any of these, existing as particles, beads, strands, precipitates, gels, sheets, tubing, spheres, containers, capillaries, pads, slices, films, plates, slides, etc. The solid support is preferably flat but may take on alternative surface configurations. For example, the solid-support may contain raised or depressed regions on which synthesis takes place. Exemplary supports include, but are not limited to, glass (including controlled-pore glass), polymerized Langmuir Blodgett films, silicone rubber, quartz, latex, polyurethane, silicon and modified silicon, Ge, gallium arsenide, GaP, silicon dioxide, silicon nitride, metals (such as gold, and other derivatizable transition metals, a variety of gels and polymers such as (poly)tetrafluoroethylene, (poly)vinylidendifluoride, polystyrene, polystyrene-divinylbenzene copolymer (e.g., for synthesis of peptides), polycarbonate, and combinations thereof. Other suitable solid support materials will be readily apparent to those of skill in the art. Solid-support base materials are generally resistant to the variety of chemical reaction conditions to which they may be subjected.

The term "oligonucleotide" refers to a polymer having at least two nucleic acid units, preferably at least about 25 nucleic acid units, more preferably at least about 40 nucleic acid units, and most preferably at least about 60 nucleic acid units.

The terms "nucleotide," "nucleic acid" and "nucleic acid unit" are used interchangeably herein and refer to both natural and unnatural nucleic acids and derivatives thereof.

The term "solid support bound oligonucleotide" refers to an oligonucleotide that is covalently bonded to a solid-support.

The term "linker" means a molecule or group of molecules attached to a substrate and spacing a synthesized polymer from the substrate for exposure/binding to a receptor.

The term "activation energy wavelength" refers to that wavelength of electromagnetic radiation that will activate a photoprotective group or photocleavable group.

The term "solid support bound nucleotide" refers to a nucleic acid or an oligonucleotide that is covalently bonded to a solid-support. In all cases, the length of nucleotide(s) on a solid-support bound nucleotide is less than the length of nucleotides on a solid-support bound oligonucleotide that is produced from the solid-support bound nucleotide.

The terms "library of oligonucleotides" and "oligonucleotide array" are used interchangeably herein and refer to a collection of oligonucleotides which are produced in a single reaction apparatus.

The term "activator" refers to a compound that facilitates coupling of one nucleic acid to another, preferably in 3'-position of one nucleic acid to 5'- position of the other nucleic acid or vice a versa.

The terms "quality," "performance" and "intensity" are used interchangeably herein when referring to oligonucleotide probes or binding of a target molecule to oligonucleotide probes mean sensitivity of oligonucleotide probes to bind to a target molecule while giving a minimum of false signals.

The terms "activated nucleoside" and "activated nucleotide" are used interchangeably herein in and refer to natural or unnatural nucleic acid monomers having a pendant activating group such as phosphite-triester, phosphotriester, H-phosphonate, or preferably phosphoramidite group on at least one of the oxygen atoms of the sugar moiety. Preferably, the activating group is on the C-3' oxygen or C-5' oxygen of the nucleic acid monomer. Typically, the activating group is on the C-3' oxygen of the nucleic acid monomer, for synthesizing probes in the 3'-5' direction, with the oligonucleotide attached to the support via the 3'-end. The activating group is on the C-5' oxygen of the nucleic acid monomer, for synthesizing probes in the 5'-3' ("reverse") direction, with the oligonucleotide attached to the support via the 5'-end.

The terms "phosphoramidite," "phosphoramidite derivative," and "amidite" are used interchangeably herein and refer to a nucleic acid having a pendent phosphoramidite group.

The term "probe" refers to a surface-immobilized nucleic acid or oligonucleotide that is recognized by a particular target by virtue of having a sequence that is complementary to the target sequence. These may also be referred to as ligands.

The term "array" refers to a preselected collection of polymers which are associated with a surface of a substrate. In a preferred embodiment of the present invention, polymers are nucleic acids or, more preferably, oligonucleotide, which are also called oligonucleotide probes. An array can include nucleic acid or oligonucleotides of a given length having all possible monomer sequences made up of a specific basis set of monomers, or a specific subset of such an array. For example, an array of all possible oligonucleotides each having 8 nucleic acids includes 65,536 different sequences. However, as noted above, a nucleic acid or oligonucleotide array also can include only a subset of the complete set of probes. Similarly, a given array can exist on more than one separate substrate, e.g., where the number of sequences necessitates a larger surface area or more than one solid substrate in order to include all of the desired oligonucleotide sequences.

The term "wafer" generally refers to a substantially flat sample of substrate (i.e., solid-support) from which a plurality of individual arrays or chips can be fabricated.

The term "functional group" means a reactive chemical moiety present on a given monomer, polymer, linker or substrate surface. Examples of functional groups include, e.g., the 3' and 5' hydroxyl groups of nucleotides and nucleosides, as well as the reactive groups on the nucleobases of the nucleic acid monomers, e.g., the exocyclic amine group of guanosine, as well as amino and carboxyl groups on amino acid monomers.

The term photoprotecting group (also called photo labile protecting groups or photogroup for short) means a material which is chemically bound to a reactive functional group on a monomer unit, linker, or polymer and which may be removed upon selective exposure to electromagnetic radiation or light, especially ultraviolet and visible light.

The term "reactive group" refers to a group that allows a covalent reaction to occur between for example a monomer and a linker or between a second monomer and a first attached monomer. A reactive group may be protected by photoprotective removable group. Removal of the photogroup, yields a deprotected reactive group.

The terms "array" and "chip" are used interchangeably herein and refer to the final product of the individual array of nucleic acid or oligonucleotide sequences, having a plurality of positionally distinct oligonucleotide sequences coupled to the surface of the substrate. "Array" is used with reference to nucleic acid or oligonucleotide, but it should be appreciated that either can be attached to a solid support. Reference will be made to oligonucleotide arrays as a preferred example of the present invention.

The term "alkyl" refers to a branched or straight chain acyclic, monovalent saturated hydrocarbon radical of one to twenty carbon atoms.

The term "alkenyl" refers to an unsaturated hydrocarbon radical which contains at least one carbon-carbon double bond and includes straight chain, branched chain and cyclic radicals.

The term "alkynyl" refers to an unsaturated hydrocarbon radical which contains at least one carbon-carbon triple bond and includes straight chain, branched chain and cyclic radicals.

The term "aryl" refers to an aromatic monovalent carboxylic radical having a single ring (e.g., phenyl) or two condensed rings (e.g., naphthyl), which can optionally be mono-, di-, or tri-substituted, independently, with alkyl, lower-alkyl, cycloalkyl, hydroxylower-alkyl, aminolower-alkyl, hydroxyl, thiol, amino, halo, nitro, lower-alkylthio, lower-alkoxy, mono-lower-alkylamino, di-lower-alkylamino, acyl, hydroxycarbonyl, lower-alkoxycarbonyl, hydroxysulfonyl, lower-alkoxysulfonyl, lower-alkylsulfonyl, lower-alkylsulfinyl, trifluoromethyl, cyano, tetrazoyl, carbamoyl, lower-alkylcarbamoyl, and di-lower-alkylcarbamoyl. Alternatively, two adjacent positions of the aromatic ring may be substituted with a methylenedioxy or ethylenedioxy group.

The term "heteroaromatic" refers to an aromatic monovalent mono- or poly-cyclic radical having at least one heteroatom within the ring, e.g., nitrogen, oxygen or sulfur, wherein the aromatic ring can optionally be mono-, di- or tri-substituted, independently, with alkyl, lower- alkyl, cycloalkyl, hydroxylower-alkyl, amino lower-alkyl, hydroxyl, thiol, amino, halo, nitro, lower-alkylthio, lower-alkoxy, mono-lower-alkylamino, di-lower-alkylamino, acyl, hydroxycarbonyl, lower-alkoxycarbonyl, hydroxysulfonyl, lower-alkoxysulfonyl, lower-alkylsulfonyl, lower-alkylsulfinyl, trifluoromethyl, cyano, tetrazoyl, carbamoyl, lower-alkylcarbamoyl, and di-lower-alkylcarbamoyl. For example, typical heteroaryl groups with one or more nitrogen atoms are tetrazoyl, pyridyl (*e*.*g*., 4-pyridyl, 3-pyridyl, 2-pyridyl), pyrrolyl (*e*.*g*., 2-pyrrolyl, 2-(N-alkyl)pyrrolyl), pyridazinyl, quinolyl (*e*.*g*. 2-quinolyl, 3-quinolyl etc.), imidazolyl, isoquinolyl, pyrazolyl, pyrazinyl, pyrimidinyl, pyridonyl or pyridazinonyl; typical oxygen heteroaryl radicals with an oxygen atom are 2-furyl, 3-furyl or benzofuranyl; typical sulfur heteroaryl radicals are thienyl, and benzothienyl; typical mixed heteroatom heteroaryl radicals are furazanyl and phenothiazinyl. Further the term also includes instances where a heteroatom within the ring has been oxidized, such as, for example, to form an N-oxide or sulfone.

The term "optionally substituted" refers to the presence or lack thereof of a substituent on the group being defined. When substitution is present the group may be mono-, di- or tri-substituted, independently, with alkyl, lower-alkyl, cycloalkyl, hydroxylower-alkyl, aminolower-alkyl, hydroxyl, thiol, amino, halo, nitro, lower-alkylthio, lower-alkoxy, mono-lower-alkylamino, di-lower-alkylamino, acyl, hydroxycarbonyl, lower-alkoxycarbonyl, hydroxysulfonyl, lower-alkoxysulfonyl, lower-alkylsulfonyl, lower-alkylsulfinyl, trifluoromethyl, cyano, tetrazoyl, carbamoyl, lower-alkylcarbamoyl, and di-lower-alkylcarbamoyl. Typically, electron-donating substituents such as alkyl, lower-alkyl, cycloalkyl, hydroxylower-alkyl, aminolower-alkyl, hydroxyl, thiol, amino, halo, lower-alkylthio, lower-alkoxy, mono-lower-alkylamino and di-lower-alkylamino are preferred.

The term "electron donating group" refers to a radical group that has a lesser affinity for electrons than a hydrogen atom would if it occupied the same position in the molecule. For example, typical electron donating groups are hydroxy, alkoxy (e.g. methoxy), amino, alkylamino and dialkylamine.

The term "leaving group" means a group capable of being displaced by a nucleophile in a chemical reaction, for example halo, nitrophenoxy, pentafluorophenoxy, alkyl sulfonates (e.g., methanesulfonate), aryl sulfonates, phosphates, sulfonic acid, sulfonic acid salts, and the like.

"Activating group" refers to those groups which, when attached to a particular functional group or reactive site, render that site more reactive toward covalent bond formation with a second functional group or reactive site. The group of activating groups which are useful for a carboxylic acid include simple ester groups and anhydrides. The ester groups include alkyl, aryl and alkenyl esters and in particular such groups as 4-nitrophenyl, N-hydroxylsuccinimide and pentafluorophenol. Other activating groups are known to those of skill in the art.

"Chemical library" or "array" is an intentionally created collection of differing molecules which can be prepared either synthetically or biosynthetically and screened for activity in a variety of different formats (e.g., libraries of soluble molecules; and libraries of compounds tethered to resin beads, silica chips, or other solid supports). The term is also intended to refer to an intentionally created collection of stereoisomers.

A "cleavable moiety" or "releasable group" refers to a molecule which can be cleaved or released under a set of distinct conditions, e.g., certain wave lengths of light of certain chemical conditions. As employed in the context of the present invention of arrays of releasable polymer the conditions much be such as not to substantially damage or harm the polymer in questions. Persons of skill in the art will recognize what cleavable moiety may be employed for example where the polymer is a nucleic acid or a peptide.

"Predefined region" refers to a localized area on a solid support. It can be where synthesis takes place or where a nucleic acid is placed. Predefined region can also be defined as a "selected region." The predefined region may have any convenient shape, e.g., circular, rectangular, elliptical, wedge-shaped, etc. For the sake of brevity herein, "predefined regions" are sometimes referred to simply as "regions." In some embodiments, a predefined region and, therefore, the area upon which each distinct compound is synthesized or placed is smaller than about 1 cm² or less than 1 mm². Within these regions, the molecule therein is preferably in a substantially pure form. In additional embodiments, a predefined region can be achieved by physically separating the regions (i.e., beads, resins, gels, etc.) into wells, trays, etc.

A "linker" is a molecule or group of molecules attached to a substrate and spacing a synthesized polymer from the substrate for exposure/binding to a receptor. "Solid support", "support", and "substrate" refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations.

Isolation and purification of the compounds and intermediates described herein can be effected, if desired, by any suitable separation or purification procedure such as, for example, filtration, extraction, crystallization, column chromatography, thin-layer chromatography, thick-layer (preparative) chromatography, distillation, or a combination of these procedures.

A "channel block" is a material having a plurality of grooves or recessed regions on a surface thereof. The grooves or recessed regions may take on a variety of geometric configurations, including but not limited to stripes, circles, serpentine paths, or the like. Channel blocks may be prepared in a variety of manners, including etching silicon blocks, molding or pressing polymers, etc.

A "monomer" is a member of the set of small molecules which can be joined together to form a polymer. The set of monomers includes but is not restricted to, for example, the set of common L-amino acids, the set of common D-amino acids, the set of synthetic amino acids, the set of nucleotides and the set of pentoses and hexoses. As used herein, monomer refers to any member of a basis set for synthesis of a polymer. Thus, monomers refers to dimmers, trimers, tetramers and higher units of molecules which can be joined to form a polymer. For example, dimmers of the 20 naturally occurring L-amino acids for a basis set of 400 monomers for synthesis of polypeptides. Different basis sets of monomers may be used at successive steps in the synthesis of a polymer. Furthermore, each of the sets may include protected members which are modified after synthesis.

A "polymer" is composed of two or more joined monomers and includes for example both linear and cyclic polymers of nucleic acids, polysaccharides, phospholipids, and peptides having either α-, β-, and ω-amino acids, hetero-polymers in which a known drug is covalently bound to any of the above, polyurethanes, polyesters, polycarbonates, polyureas, polyamides, polyethyleneimines, polyarylene sulfides, polysiloxanes, polyimides, polyacetates, or other polymers.

A "releasable group" is a moiety or chemical group which is labile, i.e., may be activated or cleaved, under a given set of conditions, but is stable under other sets of conditions.

As used in this application, the singular form "a," "an," and "the" include plural references unless the context clearly dictates otherwise. For example, the term "an agent" includes a plurality of agents, including mixtures thereof.

An individual is not limited to a human being but may also be other organisms including but not limited to mammals, plants, bacteria, or cells derived from any of the above.

The present invention has many preferred embodiments and relies on many patents, applications and other references for details known to those of the art. Therefore, when a patent, application, or other reference is cited or repeated below, it should be understood that it is incorporated by reference in its entirety for all purposes as well as for the proposition that is recited.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the example herein below. However, other equivalent conventional procedures can, of course, also be used. Such conventional techniques and descriptions can be found in standard laboratory manuals such as *Genome Analysis: A Laboratory Manual Series (Vols. I-IV), Using Antibodies: A Laboratory Manual, Cells: A Laboratory Manual, PCR Primer: A Laboratory Manual,* and Molecular *Cloning: A Laboratory Manual* (all from Cold Spring Harbor Laboratory Press), Stryer, L. (1995) *Biochemistry* (4th Ed.) Freeman, New York, *Gait, "Oligonucleotide Synthesis: A Practical Approach" 1984, IRL Press, London,* Nelson and Cox (2000), *Lehninger, Principles of Biochemistry* 3^{rd} Ed., W.H. Freeman Pub., New York, NY and Berg et al. (2002) *Biochemistry,* 5^{th} Ed., W.H. Freeman Pub., New York, NY, all of which are herein incorporated in their entirety by reference for all purposes.

The present invention can employ solid substrates, including arrays in some preferred embodiments. Methods and techniques applicable to polymer (including protein) array synthesis have been described in U.S.S.N 09/536,841, WO 00/58516, U.S. Patents Nos. 5,143,854, 5,242,974, 5,252,743, 5,324,633, 5,384,261, 5,405,783, 5,424,186, 5,451,683, 5,482,867, 5,491,074, 5,527,681, 5,550,215, 5,571,639, 5,578,832, 5,593,839, 5,599,695, 5,624,711, 5,631,734, 5,795,716, 5,831,070, 5,837,832, 5,856,101, 5,858,659, 5,936,324, 5,968,740, 5,974,164, 5,981,185, 5,981,956, 6,025,601, 6,033,860, 6,040,193, 6,090,555, 6,136,269, 6,269,846 and 6,428,752, in PCT Applications Nos. PCT/US99/00730 (International Publication Number WO 99/36760) and PCT/US01/04285, which are all incorporated herein by reference in their entirety for all purposes.

Patents that describe synthesis techniques in specific embodiments include U.S. Patents Nos. 5,412,087, 6,147,205, 6,262,216, 6,310,189, 5,889,165, and 5,959,098. Nucleic acid arrays are described in many of the above patents, but the same techniques are applied to polypeptide arrays.

Nucleic acid arrays that are useful in the present invention include those that are commercially available from Affymetrix (Santa Clara, CA) under the brand name GeneChip®. Example arrays are shown on the website at affymetrix.com.

The present invention also contemplates many uses for polymers attached to solid substrates. These uses include gene expression monitoring, profiling, library screening, genotyping and diagnostics. Gene expression monitoring, and profiling methods can be shown in U.S. Patents Nos. 5,800,992, 6,013,449, 6,020,135, 6,033,860, 6,040,138, 6,177,248 and 6,309,822. Genotyping and uses therefore are shown in USSN 60/319,253, 10/013,598, and U.S. Patents Nos. 5,856,092, 6,300,063, 5,858,659, 6,284,460, 6,361,947, 6,368,799 and 6,333,179. Other uses are embodied in U.S. Patents Nos. 5,871,928, 5,902,723, 6,045,996, 5,541,061, and 6,197,506.

The present invention also contemplates sample preparation methods in certain preferred embodiments. Prior to or concurrent with genotyping, the genomic sample may be amplified by a variety of mechanisms, some of which may employ *PCR. See, e.g., PCR Technology: Principles and Applications for DNA Amplification* (Ed. H.A. Erlich, Freeman Press, NY, NY, 1992); *PCR Protocols: A Guide to Methods and Applications* (Eds. Innis, et al., Academic Press, San Diego, CA, 1990); Mattila et al., *Nucleic Acids Res*. 19, 4967 (1991); Eckert et al., *PCR Methods and Applications 1,* 17 (1991); *PCR* (Eds. McPherson et al., IRL Press, Oxford); and U.S. Patent Nos. 4,683,202, 4,683,195, 4,800,159 4,965,188,and 5,333,675, and each of which is incorporated herein by reference in their entireties for all purposes. The sample may be amplified on the array. See, for example, U.S Patent No 6,300,070 and U.S. patent application 09/513,300, which are incorporated herein by reference.

Other suitable amplification methods include the ligase chain reaction (LCR) (*e*.*g*., Wu and Wallace, *Genomics* 4, 560 (1989), Landegren et al., *Science* 241, 1077 (1988) and Barringer et al. *Gene* 89:117 (1990)), transcription amplification (Kwoh et al., *Proc. Natl. Acad. Sci. USA* 86, 1173 (1989) and W088/10315), self-sustained sequence replication (Guatelli et al., *Proc. Nat. Acad. Sci. USA,* 87, 1874 (1990) and WO90/06995), selective amplification of target polynucleotide sequences (U.S. Patent No 6,410,276), consensus sequence primed polymerase chain reaction (CP-PCR) (U.S. Patent No 4,437,975), arbitrarily primed polymerase chain reaction (AP-PCR) (U.S. Patent No 5, 413,909, 5,861,245) and nucleic acid based sequence amplification (NABSA). (*See,* US patents nos. 5,409,818, 5,554,517, and 6,063,603, each of which is incorporated herein by reference). Other amplification methods that may be used are described in, U.S. Patent Nos. 5,242,794, 5,494,810, 4,988,617 and in USSN 09/854,317, each of which is incorporated herein by reference.

Additional methods of sample preparation and techniques for reducing the complexity of a nucleic sample are described in Dong et al., *Genome Research* 11, 1418 (2001), in U.S. Patent No 6,361,947, 6,391,592 and U.S. Patent application Nos. 09/916,135, 09/920,491, 09/910,292, and 10/013,598.

Methods for conducting polynucleotide hybridization assays have been well developed in the art. Hybridization assay procedures and conditions will vary depending on the application and are selected in accordance with the general binding methods known including those referred to in: Maniatis et al. *Molecular Cloning: A Laboratory Manual* (2^{nd} Ed. Cold Spring Harbor, N.Y, 1989); Berger and Kimmel *Methods in Enzymology*, Vol. 152, *Guide to Molecular Cloning Techniques* (Academic Press, Inc., San Diego, CA, 1987); Young and Davism, *P.N.A.S,* 80: 1194 (1983). Methods and apparatus for carrying out repeated and controlled hybridization reactions have been described in US patent 5,871,928, 5,874,219, 6,045,996 and 6,386,749, 6,391,623 each of which are incorporated herein by reference

The present invention also contemplates signal detection of hybridization between ligands in certain preferred embodiments. See U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Patent application 60/364,731 and in PCT Application PCT/US99/06097 (published as WO99/47964), each of which also is hereby incorporated by reference in its entirety for all purposes.

Methods and apparatus for signal detection and processing of intensity data are disclosed in, for example, U.S. Patents Numbers 5,143,854, 5,547,839, 5,578,832, 5,631,734, 5,800,992, 5,834,758; 5,856,092, 5,902,723, 5,936,324, 5,981,956, 6,025,641, 6,090,555, 6,141,096, 6,185,030, 6,201,639; 6,218,803; and 6,225,625, in U.S. Patent application 60/364,731 and in PCT Application PCT/US99/06097 (published as WO99/47964), each of which also is hereby incorporated by reference in its entirety for all purposes.

The practice of the present invention may also employ conventional biology methods, software and systems. Computer software products of the invention typically include computer readable medium having computer-executable instructions for performing the logic steps of the method of the invention. Suitable computer readable medium include floppy disk, CD-ROM/DVD/DVD-ROM, hard-disk drive, flash memory, ROM/RAM, magnetic tapes and etc. The computer executable instructions may be written in a suitable computer language or combination of several languages. Basic computational biology methods are described in, e.g. Setubal and Meidanis et al., *Introduction to Computational Biology Methods* (PWS Publishing Company, Boston, 1997); Salzberg, Searles, Kasif, (Ed.), *Computational Methods in Molecular Biology,* (Elsevier, Amsterdam, 1998); Rashidi and Buehler, *Bioinformatics Basics: Application in Biological Science and Medicine* (CRC Press, London, 2000) and Ouelette and Bzevanis *Bioinformatics: A Practical Guide for Analysis of Gene and Proteins* (Wiley & Sons, Inc., 2^{nd} ed., 2001).

The present invention may also make use of various computer program products and software for a variety of purposes, such as probe design, management of data, analysis, and instrument operation. See, U.S. Patent Nos. 5,593,839, 5,795,716, 5,733,729, 5,974,164, 6,066,454, 6,090,555, 6,185,561, 6,188,783, 6,223,127, 6,229,911 and 6,308,170.

Additionally, the present invention may have preferred embodiments that include methods for providing genetic information over networks such as the Internet as shown in U.S. Patent applications 10/063,559, 60/349,546, 60/376,003, 60/394,574, 60/403,381.

In accordance with one aspect of the present invention, a method is presented for releasing polymers from an array of polymers on a solid substrate, the method comprising the steps of: providing a solid substrate; attaching a plurality of linkers to the substrate, each said linker comprising a cleavable moiety, wherein said cleavable moiety is activatable only at a distinct set of conditions and wherein activation of said cleavable moiety disrupts the linker to allow release of the polymer, to provide a substrate with a plurality of attached linkers; attaching a first monomer to at least one of said plurality of attached linkers to provide an attached first monomer; attaching a second monomer to a least one of said attached first monomer or said plurality of attached linkers to provide an attached second monomor; attaching a third monomer to a least one of said attached first monomer, said second monomer or said plurality of attached linkers to provide an attached third monomer; repeating said steps of attaching monomers until the desired array of polymers is complete; and subjecting the array to the distinct set of conditions to release polymers from said array.

In preferred embodiments of this aspect of the invention, the monomers are nucleotides or amino acids. In some preferred embodiments, the cleavable moiety is a photogroup. Particularly preferred embodiments of the photogroup are selected from the group consisting of 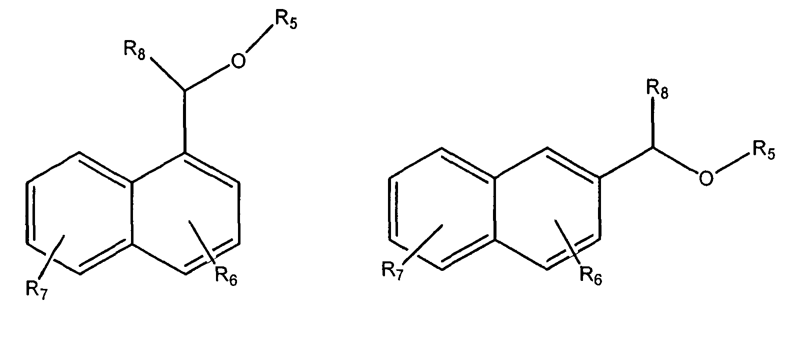 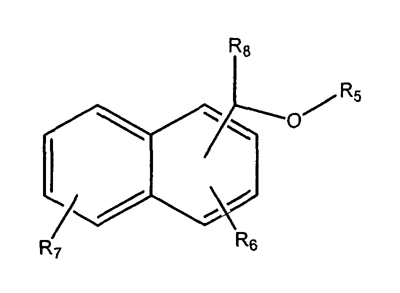 and 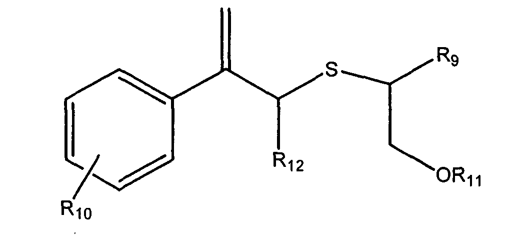 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid.

According to this aspect of the present invention, the photogroup is preferably activated by light having a wavelength of 313 nm and below. More preferably, the photogroup is activated by light having a wavelength of about 313 nm and below, but not by light having a longer wavelength than 313 nm.

In other preferred embodiments of this aspect of the present invention, the cleavable moiety is selected from the group consisting of 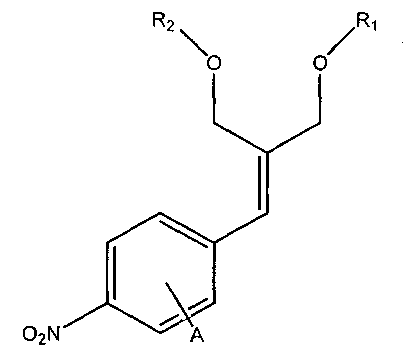 wherein R₁ is a DMT group or a photolabile protecting group, a carbonate or a phosphate, R₂ is H, a carbonate, phosphate or a thiol, A is H, a substituted alkoxy, alkyl, alkenyl, substituted aryl, amine or carboxylic acid and 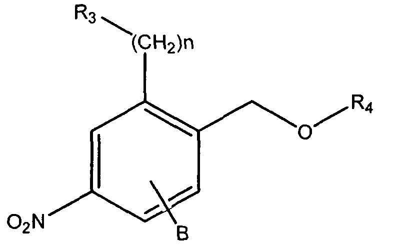 wherein R₄ is a DMT group, a carbonate, or a phosphate; R₃ is H, a carbonate, a phosphate or a thiol, and n is whole number between 0 and 6, B is H, substituted alkoxy, alkyl, alkenyl, substituted aryl, amine or carboxylic acid. Preferably, the set of conditions for release using the above compounds is a mild aqueous solution.

According to another aspect of the present invention, a method is presented for releasing linkers from an array of linkers on a solid substrate, the method having the steps of: providing a solid substrate; attaching a plurality of linkers to the solid substrate, the solid substrate having a surface, each said linker having a cleavable moiety, wherein the cleavable moiety is activatable only at a distinct set of conditions and wherein activation of the cleavable moiety disrupts the linker to allow release of the polymer from the array, to provide a plurality of attached linkers and wherein each linker has two terminal ends, the first end of which is attached to the substrate and the second end of which is away from the substrate and has a reactive group covered by a photoprotective removable group having a first activation energy wavelength; selectively exposing said photoprotective removable group on said attached linkers to light to selectively remove said photoprotective groups and provide unprotected reactive groups in one or more predefined regions; exposing under reactive conditions said one or more predefined regions with exposed reactive groups to a first monomer and attaching the first monomer to the exposed reactive groups, wherein sad first monomer comprises a reactive group protected by a photoprotective removable group having said first activation energy wavelength; selectively exposing said photoprotective removable groups on said attached linkers or said attached first monomer to light to selectively remove said photoprotective groups and expose reactive groups in one or more predefined regions; exposing under reactive conditions said one or more predefined regions with exposed reactive groups to a second monomer and attaching said second monomer to said exposed groups, wherein sad second monomer comprises a reactive group protected by a photoprotective removable group having a first activation energy wavelength; repeating said steps of selectively exposing photoprotective removable groups and exposing reactive groups to further monomers each compising a reactive group protected by a photoprotective removable group until the desired array of polymers is complete and subjecting the array to the distinct set of conditions to release the array of polymers.

In accordance with this aspect of the present invention, the monomer is preferably a nucleotide of an amino acid. Preferably, the clevable moiety comprises a photogroup having a second wavelength of activation energy, wherein the first wavelength of activation energy is different than the second wavelength of activation energy and where the cleavable moiety comprising a photogroup is not released by exposure to the first wavelength of light.

The photogroup is preferably selected from the group consisting of 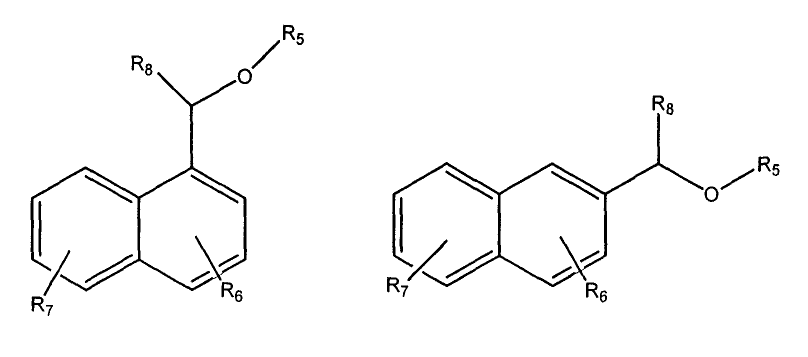 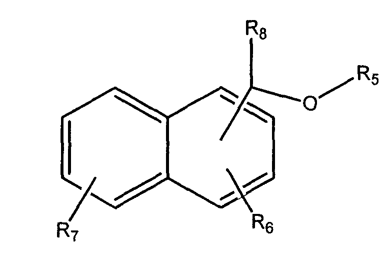 and 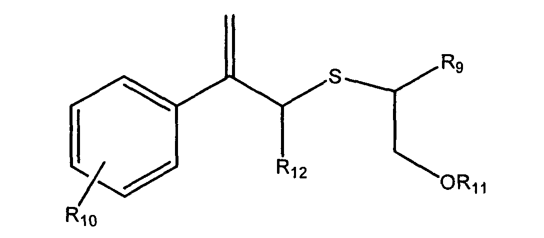 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid.

In particularly preferred embodiments of the present invention the second energy of activation wavelength is about 313 nm and below. According to another aspect of the instant invention, the cleavable moiety preferably comprises a compound selected from the group consisting of 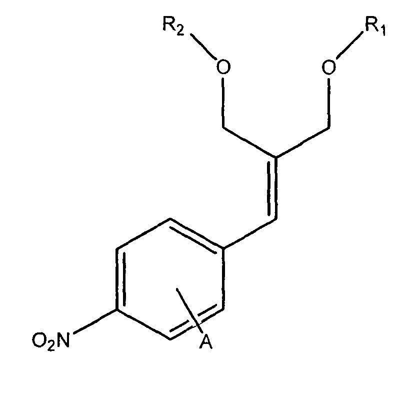 wherein R1 is a DMT group, a photolabile protective group, a carbonate or a phosphate, R2 is H, a carbonate, phosphate or a thiol, A is H, a substituted alkoxy, alkyl, alkenyl, substituted aryl, amine or carboxylic acid and 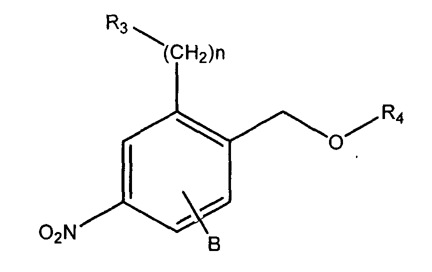 wherein R4 is a DMT group, a photolabile protecting group, a carbonate, or a phosphate; R3 is H, a carbonate, a phosphate or a thiol, and n is whole number between 0 and 6, B is H, substituted alkoxy, alkyl, alkenyl, substituted aryl, amine or carboxylic acid and wherein said set of conditions comprises a mild aqueous solution .

According to another aspect of the present invention, a releasable polymer array is presented having a solid substrate having a linker comprising a cleavable moiety which is labile under a set of conditions and attached to said linker is a polymer, wherein said polymer can be released by exposure of the array to the set of conditions. According to this aspect of the present invention, the polymer is preferably a nucleic acid, protein or peptide. More preferably, the nucleic acid is an oligonucleotide. In accordance with this aspect of the present invention, the cleavable moiety is preferably a photogroup. More preferably, the photogroup is selected from the group consisting of 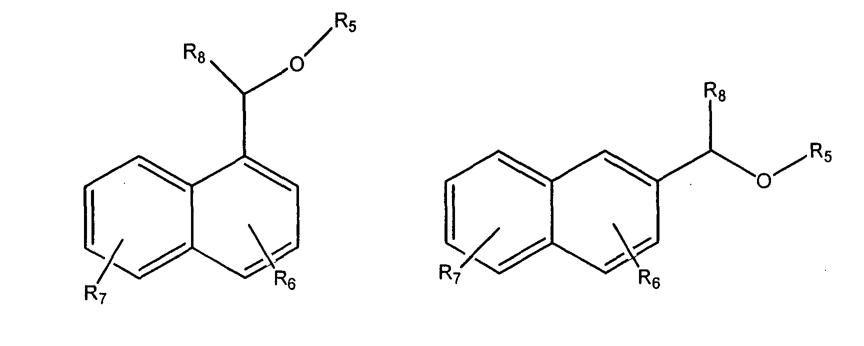 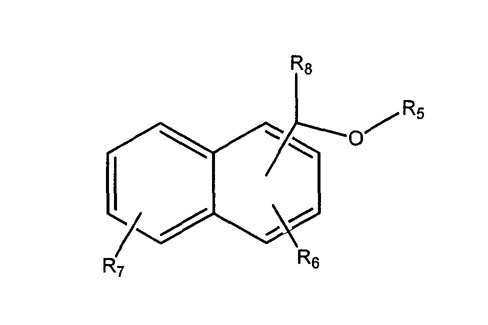 and 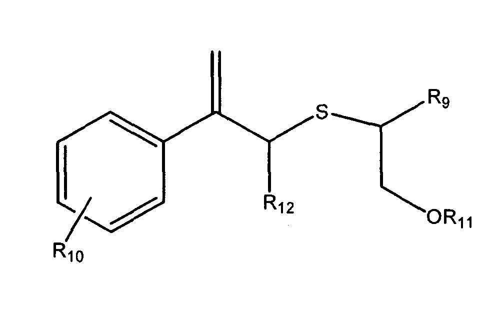 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid.

In accordance with this aspect of the present invention of the releasable polymer arrays the cleavable moiety comprises a compound selected from the group consisting of 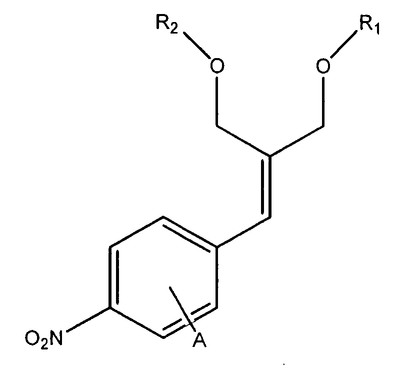 wherein R1 is a DMT group, a photolabile protective group, a carbonate or a phosphate, R2 is H, a carbonate, phosphate or a thiol, A is H, a substituted alkoxy, alkyl, alkenyl, substituted aryl, amine or carboxylic acid and 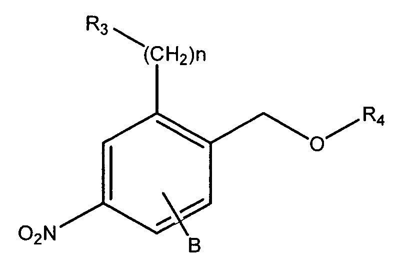 wherein R4 is a DMT group, a photolabile protecting group, a carbonate, or a phosphate; R3 is H, a carbonate, a phosphate or a thiol, and n is whole number between 0 and 6, B is H, substituted alkoxy, alkyl, alkenyl, substituted aryl, amine or carboxylic acid and wherein said set of conditions comprises a mild aqueous solution .

In accordance with another aspect of the present invention, a polymer array having releasable polymers is presented, the array having a solid substrate having attached thereto polymers, wherein one or more of said polymers has a cleavable moiety which is labile under a distinct set of conditions wherein said releasable group allows release of the polymer upon activation. Preferably, the cleavable moiety is selected from the group consisting of 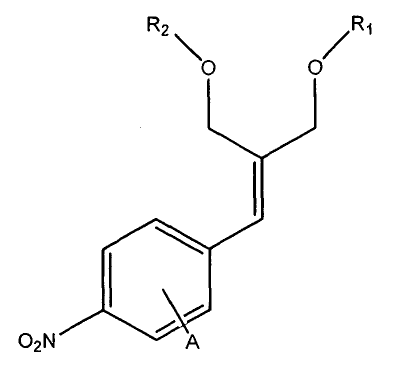 wherein R1 is a DMT group, a photolabile protective group, a carbonate or a phosphate, R2 is H, a carbonate, phosphate or a thiol, A is H, a substituted alkoxy, alkyl, alkenyl, substituted aryl, amine or carboxylic acid and 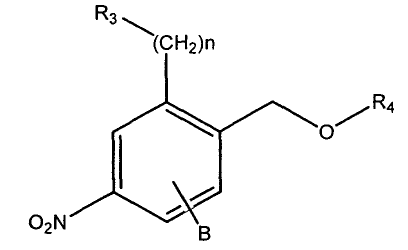 wherein R4 is a DMT group, a photolabile protecting group, a carbonate, or a phosphate; R3 is H, a carbonate, a phosphate or a thiol, and n is whole number between 0 and 6, B is H, substituted alkoxy, alkyl, alkenyl, substituted aryl, amine or carboxylic acid. The set of conditions is preferably a mild aqueous solution. It is also preferred that the cleavable moiety is preferably a photogroup. More preferably, the photogroup comprises a compound selected from the group consisting of 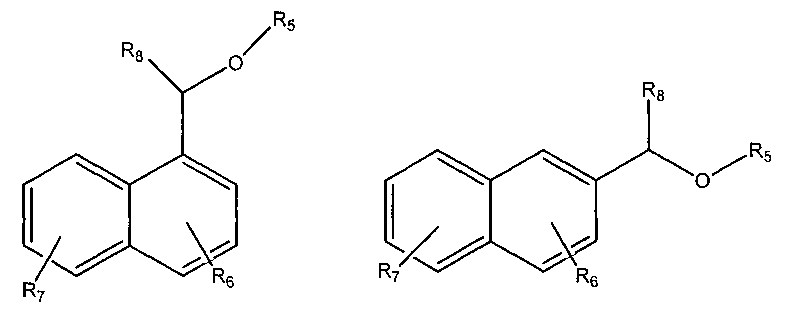 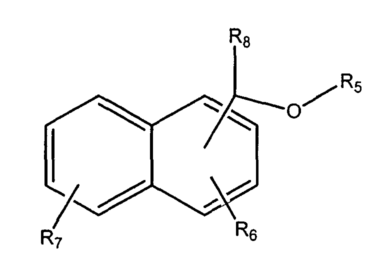 and 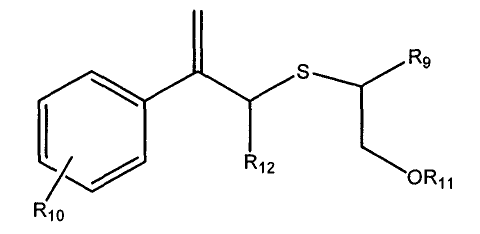 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid. In accordance with this aspect of the present invention, the polymers are preferably nucleic acids, peptides or proteins. More preferably, the polymers are oligonucleotides.

According to another aspect of the present invention, a method for fabricating a polymer array having releasable polymers is provided, the method having the steps of: providing a substrate; attaching a plurality of linkers to said substrate, said linkers comprising a cleavable moiety which is labile under a distinct set of conditions; reversibly modifying said cleavable moiety with a protecting group to provide a reversibly modified cleavable moiety wherein said modified cleavable moiety is not labile under the distinct set of conditions; attaching a first monomer to said linker; attaching a second monomer to said linker or to the first monomer; repeating said step of attaching said further monomer until the desired array of polymers is complete; and demodifying said reversibly modified releasable group.

In accordance with this aspect of the present invention, the cleavable moiety is preferably a photogroup. The protecting group is preferably 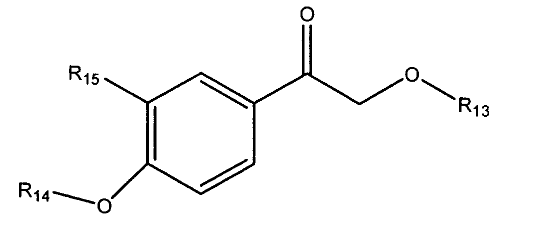 wherein R₁₃ a DMT group (4,4'dimethoxytrityl), a carbonate, or a phosphate, R₁₄ is a substituted silyl group and R₁₅ is H, or a substituted alkoxy, alkyl, alkenyl, aryl, amine or carboxcylic acid.

The linker containing the cleavable moiety should, in accordance with the present invention, be stable under conditions to which the polymer array is normally exposed. Thus, for example, in the case of a nucleic acid microarray, the linker must be stable under conditions used to fabricate the array. Thus, if the array is fabricated using spotting techniques, the releasable linker (having a cleavable moiety) must be stable under the conditions used to link the oligonucleotide to the linker. Thus, if the nucleic acid microarray is fabricated using the techniques of photolithography, the releasable linker must be stable under all conditions used during such fabrication. In addition the cleavable moiety must be stable during conditions in which the array is employed. In the case of an array of nucleic acids, the cleavable moiety must be stable to the chemicals, temperatures, conditions, etc., the array subjected to in order, for example, to detect the presence of nucleic acids, including hybridization and staining and detecting.

In one aspect of the present invention, a method for fabricating a polymer array having releasable polymers is presented, the method having the following steps (in no particular order): providing a substrate; attaching a linker to the substrate, the linker comprising a releasable group which is labile under a set of conditions; reversibly modifying the releasable group with a protecting group to provide a reversibly modified releasable group wherein the modified releasable group is not labile under the set of conditions; attaching a first monomer to the linker; attaching a second monomer to the linker or to the first monomer; repeating the step of attaching the second monomer until a polymer is provided; and demodifying the reversibly modified releasable group. In one preferred embodiment of the present invention, the releasable group comprises a photogroup.

In accordance with the present invention, conditions or sets of conditions which may be used to activate a releasable group depend upon the chemical nature of the moiety. Thus, releasable groups containing photogroups may be activated or cleaved using the appropriate wavelength of electromagnetic radiation. The releasable group, depending about its chemical nature, may alternatively be an electrochemically-sensitive group which may be cleaved in the presence of an electric field or an electric current. In still further alternative embodiments, ion beams, electron beams, or the like may be used to cleave the releasable group. In accordance with one aspect of the present invention, releasable groups may be used in conjunction with capture probes as described in U.S. App. No. 10/272,155 filed on Oct 14, 2002, incorporated here by referenced in its entirety.

With regard to the use of an electric field to activate a releasable group, alcohol groups, such as those found in nucleosides used in oligonucleotide synthesis, can for example be protected with a benzoate ester which can be electrolytically reduced to cleave the benzoate ester and reform the alcohol (Greene, et al., Protective Groups in Organic Synthesis (1991) ( incorporated here by reference). Amine groups, for example, such as those found in amino acids used for protein synthesis, can be protected with a benzyl carbamate group which can be electrolytically reduced to regenerate the amino groups. (Greene, et al.).

In one preferred embodiment of the present invention, nucleic acid probes may be released from a solid support by virtue of a releasable group through which the nucleic acid probe is connected to the solid support. In accordance with this aspect of the present invention, a releasable group must be substantially stable under the conditions used to attach the nucleic acid in question to the support, but labile, i.e., cleavable or activatable, under other conditions which are not employed to attach the nucleic acid to the solid support. The releasable group is preferably employed at the base or terminus of a nucleic acid probe to attach the probe to the solid surface such that the entire nucleic acid probe can be released upon activation or cleavage of the releasable group. Alternatively, a predetermined part of an oligonucleotide probe may be released by placement of the releasable group in positions other than the base of the probe. In accordance with the present invention, the releasable group may be attached to the nucleic acid probe at either the 5' or 3' ends.

Using lithographic methods, the photoremovable protective group is exposed to light and removed from the linker molecules in first predefined regions. As the releasable group is stable, or at least substantially stable, under these conditions it remains intact. The substrate is then washed or otherwise contacted with a first monomer which also bears the reactive group protected by a photoremovable protective group, which reacts with the exposed functional groups on the linker molecules, yielding a linker molecule, terminating in a monomer bearing the photoremovable protective group. In preferred embodiments, the monomer is an amino acid containing the photoremovable protecting group at its amino or carboxy terminus and the linker molecule terminates in an amino or carboxy acid group bearing a photoremovable protecting group. In another preferred embodiment, the monomer is a nucleotide containing the photoremovable protecting group at its 5' or 3' end and the linker molecule terminates in a 5' or 3' nucleotide bearing the photoremovable protecting group. Photoremovable protecting groups which might be employed with respect to one aspect of the present invention include methyl-6-nitropiperonyloxycarbonyl (***MeNPOC***), 6-nitrobenzyloxycarbonyl group (**NBOC**), or 6-nitroveratryloxycarbonyl group (**NVOC**) or derivatives or variants thereof.

A second set of selected regions is, thereafter, exposed to light and the photoremovable protective group on the linker molecule or monomer is removed at the second set of regions to expose functional groups. The substrate is then contacted with a second monomer for reaction with exposed functional groups. This process is repeated to selectively apply monomers until polymers of a desired length and desired chemical sequence are obtained.

In accordance with one aspect of the present invention, after fabrication of the polymers on the surface of the substrate as described above, the array may be exposed to conditions which activate the releasable group, releasing the polymer from the surface of the array. Releasing the polymer from the array may be done immediately after fabrication of the polymers is complete, i.e. before any further use is made of the array. Alternatively, the releasable polymer array may first be used for an application prior to release of the polymers. For example, where the polymers are oligonucleotides, the releasable oligonucleotide array may be used for nucleic acid analysis, including hybridization to samples of DNA or RNA prior to release. Subsequently, in accordance with one aspect of the present invention, the oligonucleotide probe, which may be hybridized to another nucleic acid, may be released from the surface of the array via activation or cleavage of the releasable group Further experimentation, such as for example sequencing, cloning, hybridization, amplification, etc., may then be performed with the released nucleic acid.

In a preferred embodiment of the present invention, photolithography is used to fabricate a releasable array of nucleic acid probes. In accordance with this aspect of the present invention, the releasable group must be substantially stable under the conditions employed in the photolithographic process, including the wavelengths of light used to deprotect the growing chains of oligonucleotides, but cleavable under other conditions not used to fabricate the array. In accordance with this aspect of the present invention, a releasable group which is activated at a shorter wavelength of radiation or light, but is stable under the longer wavelengths used in photolithography is preferred.

Herein, radiation means energy which may be selectively applied including energy having a wavelength of between 10⁻¹⁴ and 10⁴ meters including, for example, electron beam radiation, gamma radiation, x-ray radiation, ultra-violet radiation, visible light, infrared radiation, microwave radiation, and radio waves. "Irradiation" refers to the application of radiation to a surface. In accordance with one aspect of the present invention, the term light may be used to refer to all portions of the electromagnetic spectrum.

The wavelength of radiation to be employed in cleaving a releasable group containing a photogroup or moiety, in accordance with one aspect of the present invention, may be determined by determining the wavelength of light which activates the photogroup. For example, if the photo moiety is activated by ultra-violet radiation of 313 nm, light of 313 nm would be used to cleave the releasable group. The wavelength of light at which a photo moiety is activated may be determined from the literature or experimentally from techniques know to those of skill in the art.

In accordance with one aspect of the present invention, it is preferred that photo moieties employed in a releasable group are activatable at wavelengths of radiation other than 365 nm. In this regard, one photolithographic process used to produce arrays employs photoremovable protecting groups for protection of functional groups, such as hydroxyl groups, that are activated at or around 365 nm. See, e.g., U.S. Patent No. 6,261,776, incorporated here in its entirety by reference. In a preferred embodiment of the present invention, releasable groups have a very limited activation, preferably none, at 365 nm. In accordance with one aspect of the present invention, photogroups may be identified having substantially no absorbance at 365 nm, but which absorb at shorter wavelengths. Preferably, according to one aspect of the present invention, releasable groups are activated at 313 nm and below.

In accordance with one aspect of the present invention, the conditions under which a releasable group is activatable are modified or changed through reversible modification of the group to provide a reversibly protected releasable group. The reversibly protected releasable group is not activated under the conditions the unmodified releasable group could be activated at. However, in accordance with this aspect of the present invention, the reversibly protected releasable group may be rendered activatable under its normal set of conditions by reversing the modification which rendered the group non-activatable.

In a preferred embodiment of this aspect of the present invention, the releasable group is a photogroup or moiety. In accordance with the present invention, the photogroup is reversibly modified such that it is protected from photo activation at its normal activation wavelength of light. With respect to this aspect of the present invention, the photogroup can be demodified to provide a releasable group which may be activated at the photogroups normal activation wavelength.

In one aspect of the present invention, linker molecules are provided on a substrate having a surface. One end of the linker molecule is located away from the surface and another is attached to the surface of the substrate. The terminal end of the linker molecule situated away from the substrate is provided with a reactive functional group protected with a photoremovable protective group, which is removable at a wavelength of light. The linker also has a releasable group, situated in or on the linker in such a manner that the reactive functional group (or anything subsequently attached to it) is detached from the linker upon activation of the releasable group, the releasable group comprising a photogroup which is activatable with the wavelength of light. In accordance with this aspect of the present invention, the releasable group is reversibly modified to provide a reversibly protected photogroup which is substantially stable at the wavelength of light. The reversible modification of the photogroup in the releasable group may be performed at any time in accordance with the present invention. Thus, the photogroup may be modified either before or after the linker is attached to the substrate.

Using lithographic methods, the photoremovable protective group is exposed to light and removed from the linker molecules in first selected regions. The substrate is then washed or otherwise contacted with a first monomer, bearing the photoremovable protective group, that reacts with the exposed functional groups on the linker molecules, yielding a linker molecule, terminating in a monomer bearing the photoremovable protective group. In one preferred embodiment, the monomer is an amino acid containing the photoremovable protecting group at its amino or carboxy terminus and the linker molecule terminates in an amino or carboxy acid group bearing a photoremovable protecting group. In another preferred embodiment, the monomer is a nucleotide containing the photoremovable protecting group at its 5' or 3' end and the linker molecule terminates in a 5' or 3' nucleotide bearing the photoremovable protecting group. Preferably, photoremoval protecting groups which may be employed with respect to one aspect of the present invention include methyl-6-nitropiperonyloxycarbonyl (***MeNPOC***), 6-nitrobenzyloxycarbonyl group (**NBOC**), or 6-nitroveratryloxycarbonyl group (**NVOC**) or derivatives or variants thereof as appropriate.

A second set of selected regions is, thereafter, exposed to light and the photoremovable protective group on the linker molecule or monomer is removed at the second set of regions. The substrate is then contacted with a second monomer for reaction with exposed functional groups. This process is repeated to selectively apply monomers until polymers of a desired length and desired chemical sequence are obtained.

In accordance with one aspect of the present invention, after fabrication of the polymers on the surface of the substrate as described above, the reversibly protected releasable group is exposed to conditions which reverse the modification to the releasable group. The array may then be exposed to conditions which activate the releasable group, releasing the polymer from the surface of the array. Activation of the release group may be performed either before or after the array has been used in an application. In a preferred embodiment of the present invention, the monomers are nucleotides and the polymers are oligonucleotides. This oligonucleotide array may be used for nucleic acid analysis, including hybridization to samples of DNA or RNA. Subsequently, in accordance with one preferred embodiment, the oligonucleotide probe, which may be hybridized to another nucleic acid, may be released from the surface of the array via activation or cleavage of the releasable group as set forth above. Further experimentation, such as for example sequencing, cloning, hybridization, amplification, etc., may then be performed with the released nucleic acid.

In accordance with this aspect of the present invention, standard photo protecting groups such as MeNPOC, NBOC, or NVOC may be incorporated into a releasable group and reversibly modified to provide protected MeNPOC, NBOC, or NVOC groups to prevent their normal photoactivation at or around 365 nm. In accordance with this aspect of the present invention, the same photogroup may be used in the releasable group as is employed to protect the hydroxyl groups of the growing oligonucleotide chain.

The appropriate demodification chemical environment may be determined by a person of ordinary skill based on the disclosures herein and the chemistry of the group used to modify or protect the releasable group. In accordance with this aspect of the present invention, it is important that the demodification chemical environment does not adversely affect the nucleic acid array. Suitable conditions for demodification may be determined by those of ordinary skill based on the disclosures herein, the chemistry of the protecting group used to modify the releasable group and the stabilities of the various bonds in the polymer array under different chemical conditions.

In some embodiments of the present invention, a plurality of different releasable groups or reversibly protected releasable groups may be employed on a polymer array such that predetermined polymers may be released by chosen conditions. For example, in accordance with this aspect of the present invention, a plurality of different releasable groups, comprising photogroups, having different patterns of photoactivation, may be employed at predetermined locations of a nucleic acid array to allow release of preselected nucleic acid probes at different wavelengths of light. Alternatively, an electrically activated releasable group may be provided in some locations of an array and a releasable group comprising a photogroup may be employed in others to provide for selective release of polymers on the surface of the array.

### EXAMPLES

In one aspect of the present invention, releasable polymers may be represented by the formula:

S-Y-X I

In formula I, X is a releasable polymer, i.e. a polymer composed of monomers, which is attached to the Y group, which is a linker. The attachment, in accordance with the present invention, is preferably by chemical means such as covalent or ionic binding. In other preferred embodiments the attachment is by moleculer recognition such as an antigen-antibody or biotin-streptavidin.

Y is a linker which is generally stable but labile or cleavable under a given set of conditions and which attaches the releasable polymer to a solid substrate. The releasable polymer is stably bound to the substrate (S) through the linker (Y) until the linker is cleaved by exposure to the cleavage conditions (a distinct set of conditions which activate the releasable group or cleavable moiety, which causes release of the releasable polymer. In a preferred embodiment of the present invention the cleavage conditions comprise a mild reactive aqueous solution.

One particularly preferred class of linkers (Y) which are postulated to be cleaved by mild reactive aqueous conditions is represented by formula II

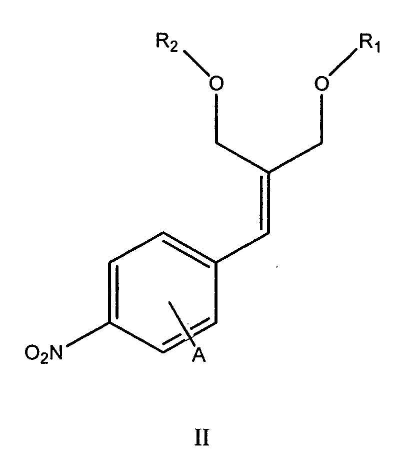

In formula II, R₁ is a DMT group, a photolabilte protecting group, a carbonate or a phosphate, R₂ is H, a carbonate, phosphate or a thiol, A is H, a substituted alkoxy, alkyl, alkenyl, substituted aryl, amine or carboxylic acid.

In another preferred embodiment of the present invention, Y is represented by formula III 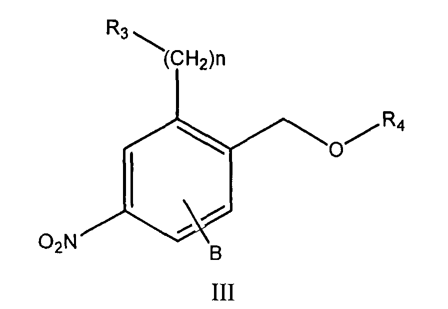 wherein R₄ is a DMT group, a photolabile protecting group, a carbonate, or a phosphate; R₃ is H, a carbonate, a phosphate or a thiol, and n is whole number between 0 and 6, B is H, substituted alkoxy, alkyl, alkenyl, substituted aryl, amine or carboxylic acid.

In accordance with one aspect of the present invention, the release of the polymers from the substrate is believed to be achieved according to scheme 1

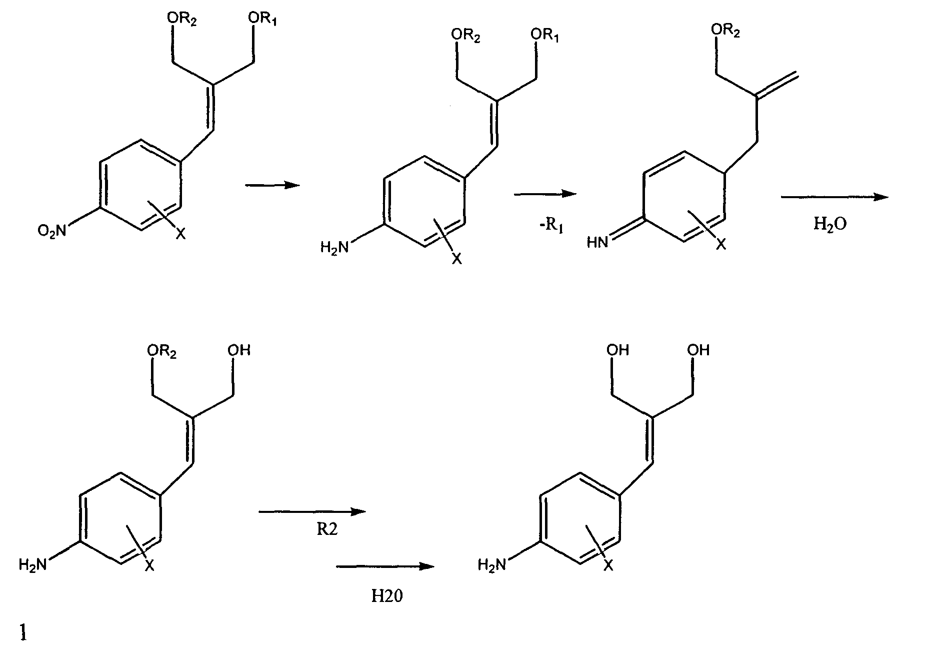

In scheme 1, according to one aspect of the instant invention, the linker comprises a compound according to formula II, set forth above, and is connected to the support (S) through the moiety R2. The presence of DMT or a photolabile protecting functionality on R1 allows for polymer synthesis (the polymer is preferably DNA or RNA) on a solid support, for example an appropriately prepared glass slide or chip. After synthesis of the polymer and/or hybridization of the polymer to a binding agent, the polymer, also referred to as a probe, can be removed by addition by exposing it to a mild aqueous solution. In accordance with one aspect of the present invention, the aqueous solution is preferably an aqueous solution of a mild organic acid in the presence of a metal. Preferably, the mild organic acid is acetic acid and the metal is zinc. The mild aqueous solution is sufficient to transform the nitro group into an amino a group which releases the polymer (e.g. the nucleic acid) via an electro-cyclic reaction as depicted in scheme 1.

References relating to the above polymers releasable under mild aqueous solution include the following:
1. Carl, P.L., Charkravarty, P.K., Katzenellenbogen, J.A. *J.Med.Chem*. **1981**, 24(5), 479-80.
2. deGroot, F.M.H., Loos, W.J., Koekkoek, R., van BerKom, L.W.A., Busscher, G.F., Seelen, A.E, Albrecht, C., de Bruijn, P., and Scheeren, H.W. *J.Org.Chem.* **2001**, 66(26), 8815-30
3. deGroot, F.M.H., Albrecht, C., Koekkoek, R., Beusker, P.H., and Scheeren, H.W. *Angew.Chem.Int.Ed.* **2003**, 42, 4490-94
each of which is hereby incorporated be references for all purposes.

In accordance with another aspect of the present invention, it is preferred that the linker comprises a photogroup which may be selected from the group of formulas set forth below: 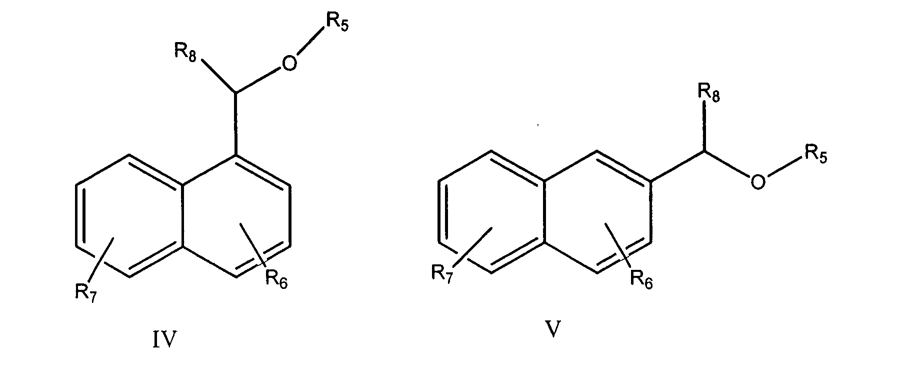 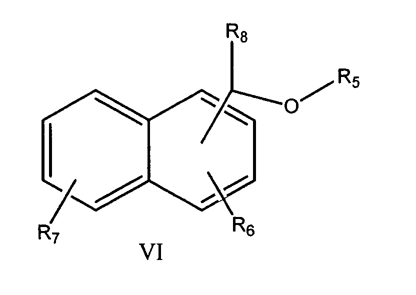 and

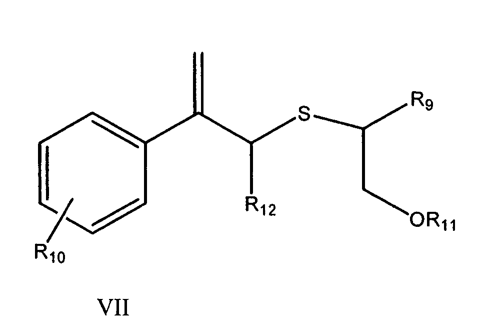

In accordance with yet another aspect of the present invention, it is preferred that the linker group Y comprises a photogroup which can be reversibly modified with a chemical moiety of the structure:

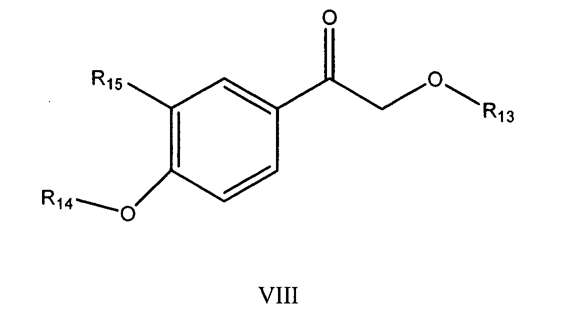

After fabrication of the polymer array, group VIII can be removed, for example by reaction with a solution of TBAF (tetrabutylammonium fluoride).

In accordance with this aspect of the present invention, the groups disclosed above may be functionalized to be used in a DNA synthesizer as DMT/phosphoramidite derivatives.

As used above, R₅, R₁₁ and R₁₃ are, independently, a DMT group (4,4'dimethoxytrityl), a carbonate, or a phosphate. R₈, R₉ and R₁₂ are, independently H, alkly, alkenyl, or substituted aryl. R₆, R₇, R₁₀, and R₁₅ are, independently, H, or a substituted alkoxy, alkyl, alkenyl, aryl, amine or carboxcylic acid. R₁₄ is a substituted silyl group.

Reference literature on the mechanism of photolysis include the following references: DeCosta, D.P. and Pincock, J.A. J.Am.Chem.Soc. 1989, 111, 8948-8950; DeCosta, D.P. and Pincock, J.A. J.Am.Chem.Soc. 1993, 115, 2180-2190; and Givens R.S. and Matuszewski B., J. Am.Chem.Soc. 1984, 6860-6861.

Styryl thioethers compounds are described, inter alia, in Fox, M.A. and Tribel, C.A. J.Org.Chem. 1983, 48, 835-840. p-hydroxyphenacil compounds are described, inter alia, in Zhang, L., Corrie, J.E.T., Ranjit, V., Munashinghe, N., Wan, P., J.Am.Chem.Soc. 1999, 121, 5625-5632.

Each of the above references is incorporated here by reference for all purposes.

According to another aspect of the present invention, compounds are provided which are useful for providing releasable polymers. In particularly preferred embodiments of the present invention, the releasable polymers are selected from the group consisting of nucleic acids and peptides.

According to one aspect of the present invention, releasable polymers may be employed in the use of high density molecular arrays on solid supports. Releasable polymers in accordance with one aspect of the present invention comprise a linker group, having a masked reactive sight such that the linker group is generally stable, but can be removed or cleaved by exposure to particular conditions. Preferably such conditions comprise, exposure to a mild reactive aqueous solution. In particularly preferred embodiments of the instant invention, mild reactive aqueous solutions include an aqueous solution of acetic acid in the presence of a metal. Non-limiting examples of metal include zinc (Zn).

## Claims

**1.** A method for releasing polymers from an array of polymers on a solid substrate, said method comprising the steps of:
providing a solid substrate;
attaching a plurality of linkers to the substrate, each said linker comprising a cleavable moiety, wherein said cleavable moiety is activatable only at a distinct set of conditions and wherein activation of said cleavable moiety disrupts the linker to allow release of the polymer, to provide a substrate with a plurality of attached linkers;
attaching a first monomer to at least one of said plurality of attached linkers to provide an attached first monomer;
attaching a second monomer to a least one of said attached first monomer or said plurality of attached linkers to provide an attached second monomor;
attaching a third monomer to a least one of said attached first monomer, said second monomer or said plurality of attached linkers to provide an attached third monomer;
repeating said steps of attaching monomers until the desired array of polymers is complete;
and subjecting the array to the distinct set of conditions to release polymers from said array.

**2.** The method of claim 1 wherein said monomers are nucleotides.

**3.** The method of claim 1 wherein said cleavable moiety comprises a photogroup.

**4.** The method of claim 3 wherein said photogroup is selected from the group consisting of 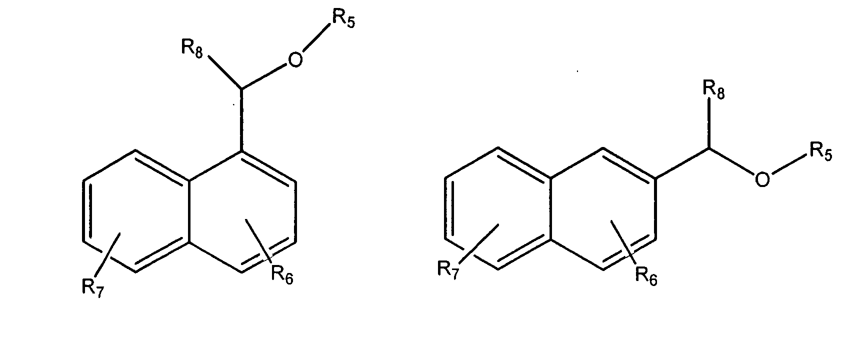 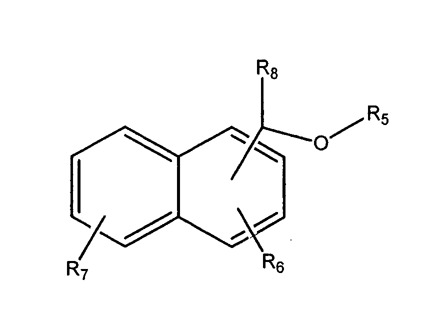 and 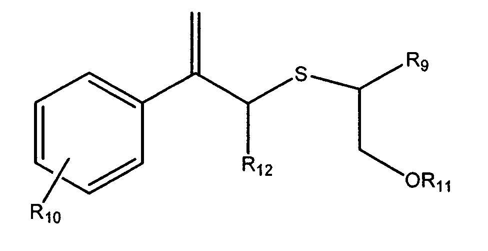 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid.

**2.** The method of claim 3 wherein said photogroup is activated by light having a wavelength of 313 nm and below.

**3.** The method of claim 3 wherein said photogroup is activated by light having a wavelength of about 313 nm and below, but not above 313 nm.

**6.** The method of claim 1 wherein said monomers are amino acids.

**7.** The method of claim 1 wherein said cleavable moiety is selected from the group consisting of 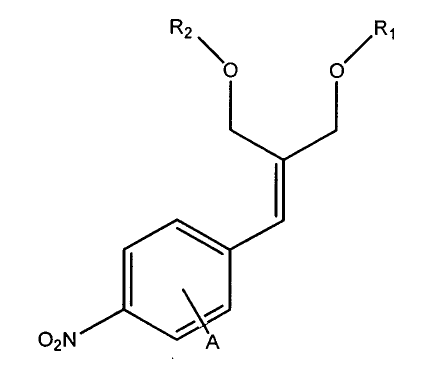 wherein R₁ is a DMT group or a photolabile protecting group, a carbonate or a phosphate, R₂ is H, a carbonate, phosphate or a thiol, A is H, a substituted alkoxy, alkyl, alkenyl, substituted aryl, amine or carboxylic acid and 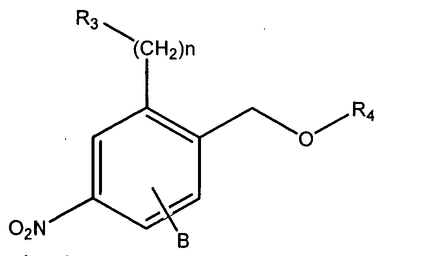 wherein R₄ is a DMT group, a carbonate, or a phosphate; R₃ is H, a carbonate, a phosphate or a thiol, and n is whole number between 0 and 6, B is H, substituted alkoxy, alkyl, alkenyl, substituted aryl, amine or carboxylic acid
and wherein said set of conditions comprises a mild aqueous solution .

**8.** A method for releasing polymers from an array of polymers on a solid substrate, said method comprising the steps of:
providing a solid substrate;
attaching a plurality of linkers to said solid substrate, said solid substrate having a surface, each said linker comprising a cleavable moiety, wherein said cleavable moiety is activatable only at a distinct set of conditions and wherein activation of said cleavable moiety disrupts the linker to allow release of the polymer from the array, to provide a plurality of attached linkers and wherein each said linker has two terminal ends, the first end of which is attached to the substrate and the second end of which is away from the substrate and comprises a reactive group covered by a photoprotective removable group having a first activation energy wavelength;
selectively exposing said photoprotective removable group on said attached linkers to light to selectively remove said photoprotective groups and provide unprotected reactive groups in one or more predefined regions;
exposing under reactive conditions said one or more predefined regions with exposed reactive groups to a first monomer and attaching said first monomer to the exposed reactive groups, wherein sad first monomer comprises a reactive group protected by a photoprotective removable group having said first activation energy wavelength;
selectively exposing said photoprotective removable groups on said attached linkers or said attached first monomer to light to selectively remove said photoprotective groups and expose reactive groups in one or more predefined regions;
exposing under reactive conditions said one or more predefined regions with exposed reactive groups to a second monomer and attaching said second monomer to said exposed groups, wherein sad second monomer comprises a reactive group protected by a photoprotective removable group having a first activation energy wavelength;
repeating said steps of selectively exposing photoprotective removable groups and exposing reactive groups to further monomers each compising a reactive group protected by a photoprotective removable group until the desired array of polymers is complete and
subjecting the array to the distinct set of conditions to release the array of polymers.

**9.** A method for releasing polymers from an array of polymers on a solid substrate according to claim 8 wherein said monomer is a nucleotide.

**10.** A method for releasing polymers from an array of polymers on a solid substrate according to claim 8 wherein said clevable moiety comprises a photogroup having a second wavelength of activation energy, wherein said first wavelength of activation energy is different than said second wavelength of activation energy and where said cleavable moiety comprising a photogroup is not released by exposure to said first wavelength of light.

**11.** A method for releasing polymers from an array of polymers on a solid substrate according to claim 10 wherein said photogroup is selected from the group consisting of 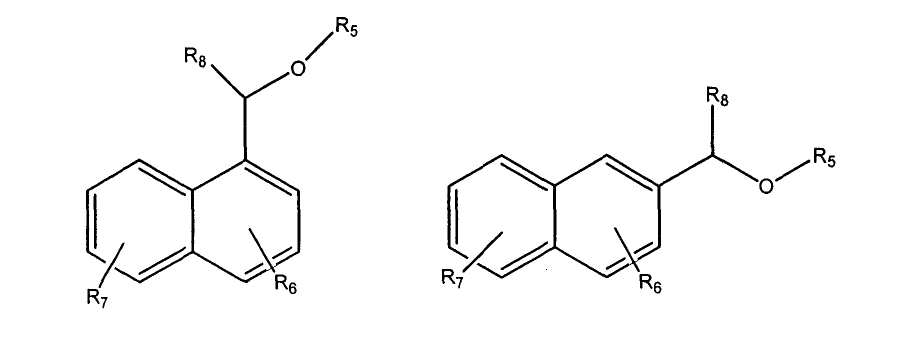 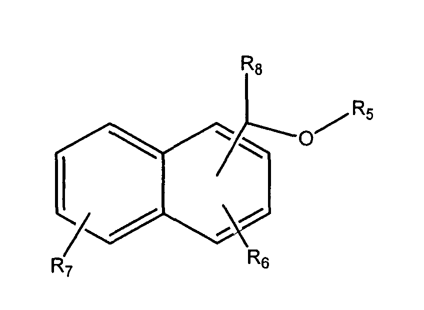 and 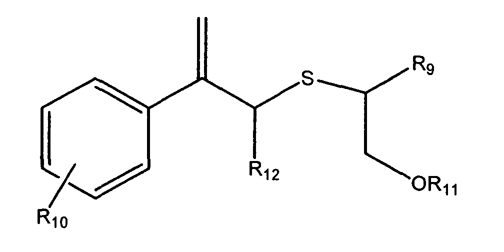 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid.

**12.** A method for releasing polymers from an array of polymers on a solid substrate according to claim 10 wherein said second energy of activation wavelength is about 313 nm and below.

**13.** A method for releasing polymers from an array of polymers on a solid substrate according to claim 8 wherein said monomer is an amino acid.

**14.** A method for releasing polymers from an array of polymers on a solid substrate according to claim 8 wherein said cleavable moiety comprises a compound selected from the group consisting of 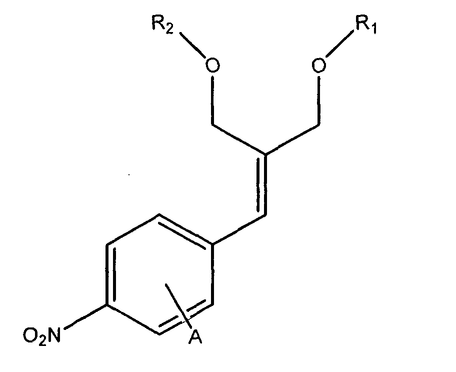 wherein R1 is a DMT group, a photolabile protective group, a carbonate or a phosphate, R2 is H, a carbonate, phosphate or a thiol, A is H, a substituted alkoxy, alkyl, alkenyl, substituted aryl, amine or carboxylic acid and 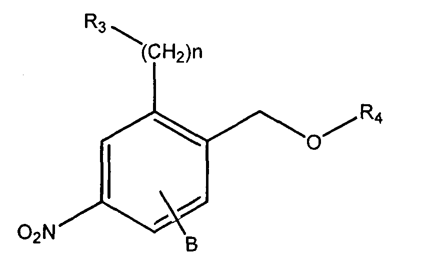 wherein R4 is a DMT group, a photolabile protecting group, a carbonate, or a phosphate; R3 is H, a carbonate, a phosphate or a thiol, and n is whole number between 0 and 6, B is H, substituted alkoxy, alkyl, alkenyl, substituted aryl, amine or carboxylic acid and wherein said set of conditions comprises a mild aqueous solution .

**15.** A releasable polymer array comprising a substrate having a linker comprising a cleavable moiety which is labile under a set of conditions and attached to said linker is a polymer, wherein said polymer can be released by exposure of the array to the set of conditions.

**16.** A releasable polymer array according to claim 15 wherein said polymer is a nucleic acid.

**17.** A releasable polymer array according to claim 16 wherein said nucleic acid is an oligonucleotide.

**18.** A releasable polymer array according to claim 15 wherein said cleavable moiety comprises a photogroup.

**19.** A releasable polymer array according to claim 18 wherein said photogroup is selected from the group consisting of 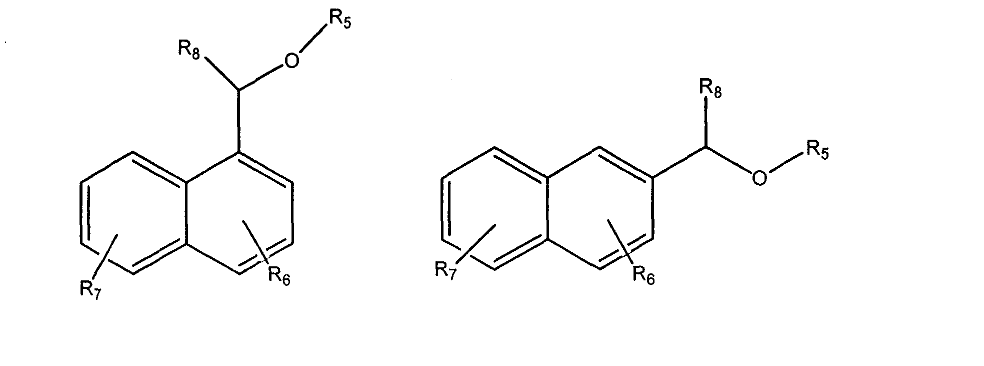 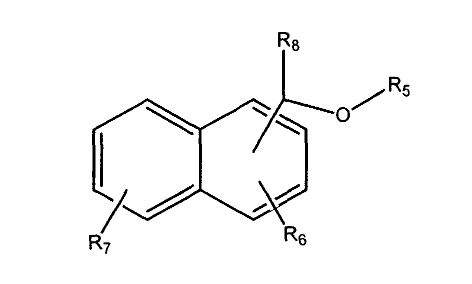 and 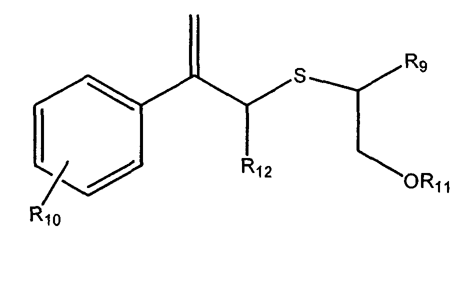 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid.

**20.** A releasable polymer array according to claim 18 wherein said polymer is a peptide.

**21.** A releasable polymer arrays according to claim 18 wherein said cleavable moiety comprises a compound selected from the group consisting of 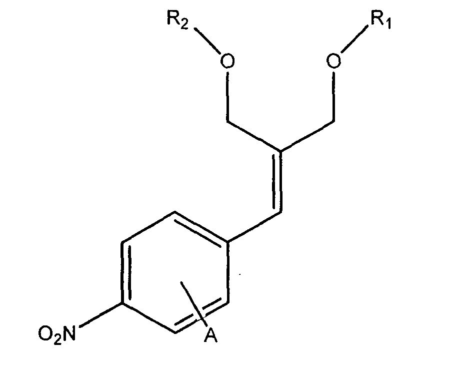 wherein R1 is a DMT group, a photolabile protective group, a carbonate or a phosphate, R2 is H, a carbonate, phosphate or a thiol, A is H, a substituted alkoxy, alkyl, alkenyl, substituted aryl, amine or carboxylic acid and 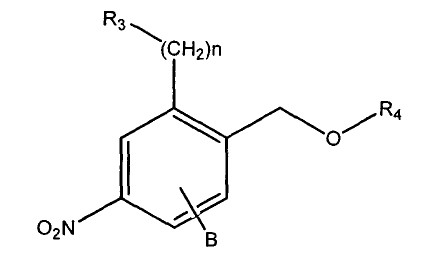 wherein R4 is a DMT group, a photolabile protecting group, a carbonate, or a phosphate; R3 is H, a carbonate, a phosphate or a thiol, and n is whole number between 0 and 6, B is H, substituted alkoxy, alkyl, alkenyl, substituted aryl, amine or carboxylic acid
and wherein said set of conditions comprises a mild aqueous solution .

**22.** A polymer array having releasable polymers, said array comprising a substrate having attached thereto polymers, wherein one or more of said polymers comprises a cleavable moiety which is labile under a distinct set of conditions wherein said releasable group allows release of the polymer upon activation.

**23.** A nucleic acid array according to claim 22 wherein said cleavable moiety comprises a photogroup which may be activated by light having a wavelength of 313 nm and below.

**24.** A nucleic acid array according to claim 22 wherein said cleavable moiety is photogroup comprises a compound selected from the group consisting of 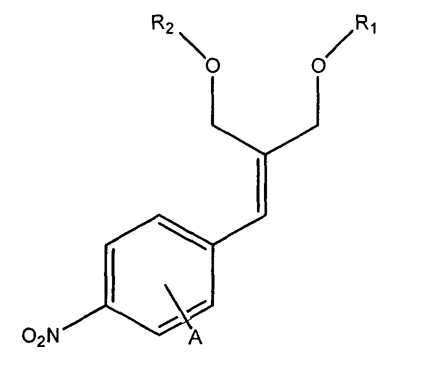 wherein R1 is a DMT group, a photolabile protective group, a carbonate or a phosphate, R2 is H, a carbonate, phosphate or a thiol, A is H, a substituted alkoxy, alkyl, alkenyl, substituted aryl, amine or carboxylic acid and 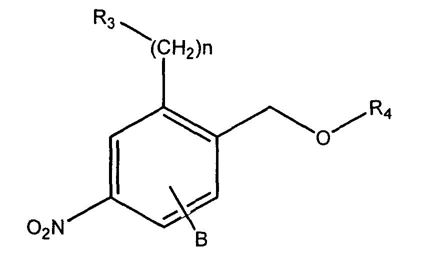 wherein R4 is a DMT group, a photolabile protecting group, a carbonate, or a phosphate; R3 is H, a carbonate, a phosphate or a thiol, and n is whole number between 0 and 6, B is H, substituted alkoxy, alkyl, alkenyl, substituted aryl, amine or carboxylic acid
and wherein said set of conditions comprises a mild aqueous solution.

**25.** A nucleic acid array according to claim 22 wherein said cleavable moiety is a photogroup comprises a compound selected from the group consisting of 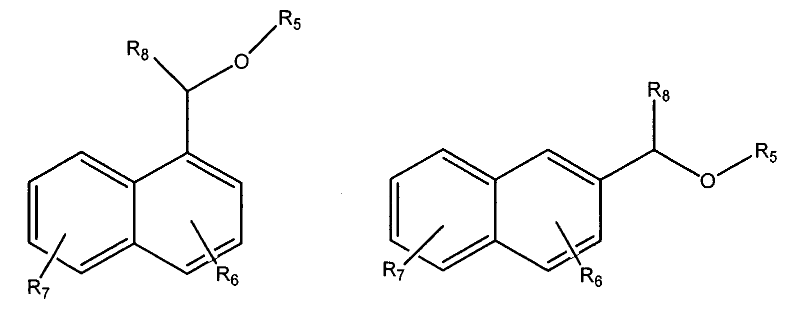 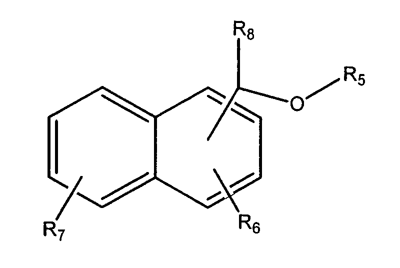 and 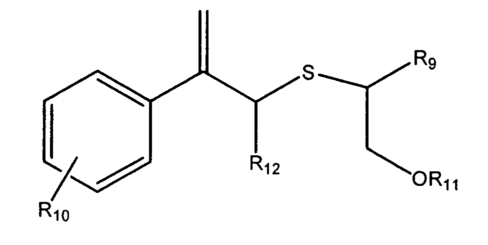 wherein R₅ and R₁₁ are, independently, a DMT group (4,4'dimethoxytrityl), a carbonate, or a phosphate, R₈, R₉ and R₁₂ are, independently H, alkly, alkenyl, or substituted aryl, and R₆, R₇, and R₁₀ are, independently, H, or a substituted alkoxy, alkyl, alkenyl, aryl, amine or carboxcylic acid.

**26.** A polymer array having releasable polymers according to claim 22 wherein said polymers are nucleic acids.

**27.** A polymer array having releasable polymers according to claim 26 wherein said nucleic acids are oligonucleotides.

**28.** A polymer array having releasable polymers according to claim 22 wherein said polymers are selected from the group consisting of proteins and peptides.

**29.** A method for fabricating a polymer array having releasable polymers, said method comprising the steps of:
providing a substrate;
attaching a plurality of linkers to said substrate, said linkers comprising a cleavable moiety which is labile under a distinct set of conditions;
reversibly modifying said cleavable moiety with a protecting group to provide a reversibly modified cleavable moiety wherein said modified cleavable moiety is not labile under the distinct set of conditions;
attaching a first monomer to said linker;
attaching a second monomer to said linker or to the first monomer;
repeating said step of attaching said further monomer until the desired array of polymers is complete; and
demodifying said reversibly modified releasable group.

**30.** A method for fabricating a polymer array according to claim 29 wherein said cleavable moiety comprises a photogroup.

**31.** A method for fabricating a polymer array according to claim 30 wherein said protecting group is 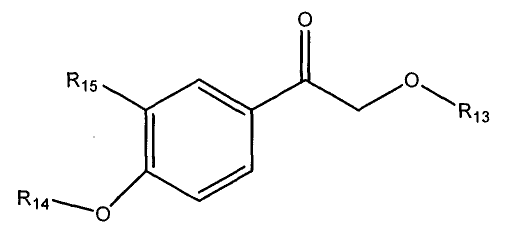 wherein R₁₃ a DMT group (4,4'dimethoxytrityl), a carbonate, or a phosphate, R₁₄ is a substituted silyl group and R₁₅ is H, or a substituted alkoxy, alkyl, alkenyl, aryl, amine or carboxcylic acid.
